# EUROPEAN PATENT SPECIFICATION

(11) **EP 2 812 351 B1**
(45) Date of publication and mention of the grant of the patent: **28.03.2018**
(21) Application number: 13710308.1
(22) Date of filing: 08.02.2013
(51) Int. Cl.: C07K 14/435, C07K 14/47, A61K 39/00, A61K 9/00, A61K 9/70, A61K 38/17

(54) **PHARMACEUTICAL COMPOSITION FOR THE TREATMENT OF MULTIPLE SCLEROSIS**
PHARMAZEUTISCHE ZUSAMMENSETZUNG ZUR BEHANDLUNG VON MULTIPLER SKLEROSE
COMPOSITION PHARMACEUTIQUE DESTINÉE AU TRAITEMENT DE LA SCLÉROSE MULTIPLE

(30) Priority: 10.02.2012 PL 39807712
(43) Date of publication of application: 17.12.2014
(73) Proprietor: SELMAJ, Krzysztof, 90-158 Lodz (PL); Szczepanik, Marian, 31-566 Kraków (PL)
(72) Inventor: SELMAJ, Krzysztof, 90-158 Lodz (PL); Szczepanik, Marian, 31-566 Kraków (PL)
(74) Representative: Witek, Rafal
(86) International application number: PCT/EP2013/052615
(87) International publication number: WO 2013/117742

(56) References cited:
- WO-A2-2007/131210
- WO-A2-2009/093143
- MACIEJ JURYNCZYK ET AL: "Immune regulation of multiple sclerosis by transdermally applied myelin peptides", ANNALS OF NEUROLOGY, vol. 68, no. 5, 29 November 2010 (2010-11-29), pages 593-601, XP055092292, ISSN: 0364-5134, DOI: 10.1002/ana.22219
- AGATA WALCZAK ET AL: "Clinical and MRI Assessment of Transdermally Applied Myelin Peptides in MS Patients", NEUROLOGY, vol. 76 (Suppl 4), 1 March 2011 (2011-03-01), page A546, XP009174950,
- M SZCZEPANIK: "Mechanisms of immunological tolerance to the antigens of the central nervous system. Skin-induced tolerance as a new therapeutic concept", JOURNAL OF PHYSIOLOGY AND PHARMACOLOGY : AN OFFICIAL JOURNAL OF THE POLISH PHYSIOLOGICAL SOCIETY, vol. 62, no. 2, April 2011 (2011-04), pages 159-165, XP055092298, Poland
- John Gever: "AAN: Skin Patch Induces MS Immune Tolerance", , 15 April 2011 (2011-04-15), XP055092323, Retrieved from the Internet: URL:http://www.medpagetoday.com/MeetingCov erage/AAN/25963 [retrieved on 2013-12-09]

### SEQUENCE LISTING

<110> Selmaj, Krzysztof Szczepanik, Marian
<120> Pharmaceutical composition for the treatment of multiple sclerosis.
<130> PZ/1978/RW/PCT
<150> PLP396077
   <151> 2012-02-10
<160> 3
<170> PatentIn version 3.5
<210> 1
   <211> 15
   <212> PRT
   <213> artificial
<220>
   <223> MBP peptide
<400> 1
   Val His Phe Phe Lys Asn Ile Val Thr Pro Arg Thr Pro Pro Pro
   1               5                   10                  15
<210> 2
   <211> 21
   <212> PRT
   <213> artificial
<220>
   <223> MOG peptide
<400> 2
   Met Glu Val Gly Trp Tyr Arg Ser Pro Phe Ser Arg Val Val His Leu
   1               5                   10                  15
   Tyr Arg Asn Gly Lys
               20
<210> 3
   <211> 13
   <212> PRT
   <213> artificial
<220>
   <223> PLP peptide
<400> 3
   His Ser Leu Gly Lys Trp Leu Gly His Pro Asp Lys Phe
   1               5                   10

## Description

The subject of the present invention is a pharmaceutical composition for use in the treatment of multiple sclerosis, in a form designed for topical administration.

Myelin proteins are components of myelin, the substance which forms the sheath around axial fibres in the nervous system. The pathogenesis of MS is based on the degradation of myelin caused by the activity of autoreactive lymphocytes against antigens contained in myelin protein peptides. Patent description No. 204536 discloses the use of a single antigen in the form of guinea pig myelin basic protein (GPMBP) for preventing and treating multiple sclerosis.

Maciej Jurynczyk et al. "Immune regulation of multiple sclerosis by transdermally applied myelin peptides", Annals of Neurology, vol. 68, no. 5, 2010, Agata Walczak et al. "Clinical and MRI Assessment of Transdermally Applied Myelin Peptides in MS Patients", Neurology, vol. 76, 2011 and WO 2009/093143 disclose a composition composed of peptides selected from a group encompassing: a peptide of the *myelin basic protein -* MBP, human protein sequence available in GeneBank: Acc. Number: CAG46717; GI: 49456793), a peptide from *myelin oligodendrocytes glycoprotein -* MOG, the human protein sequence is available from GenBank: Acc. Number: CAQ08215; GI: 168984894) as well as a peptide from the human *proteolipid protein -* PLP, the human protein sequence is available from GenBank: Acc. Number: NP_001122306; GI: 192449447), for use in the treatment of multiple sclerosis in a form designed for topical administration.

As a peptide from a basic myelin protein one should understand a peptide containing at least 5 amino-acids from the region between amino-acids 88 and 102 of this protein, preferably exhibiting at least 80% homology to the region between amino-acids 88 and 102 of the human myelin basic protein, particularly preferably this may be an MBP peptide with the sequence: H₂N- VHF FKN IVT PRT PPP - COOH (Seq. ID. No. 1).

As a peptide derived from myelin protein and oligodendrocytes one should understand a peptide containing at least 5 amino-acids from the region between amino-acids 34 and 54 of this protein, preferably exhibiting at least 80% homology to the region between amino-acids 34 and 54 of the human myelin basic protein, particularly preferably this may be the MOG peptide with the sequence: H₂N - MEV GWY RSP FSR VVH LYR NGK - COOH (Seq. ID. No. 2).

As a peptide derived from proteolipid protein one should understand a peptide containing at least 5 amino-acids from the region between amino-acids 140 and 152 of this protein, preferably exhibiting at least 80% homology to the region between amino-acids 140 and 152 of the human myelin basic protein, particularly preferably this may be a PLP peptide with the sequence: H₂N- HSL GKW LGH PDK F - COOH (Seq. ID. No. 3).

However, none of the documents belonging to the prior art describes the possibility to obtain the decrease of the rate of progression of multiple sclerosis over the year following the end of treatment.

The subject of the present invention is a pharmaceutical composition consisting of: a MBP peptide with the Seq. ID No. 1, a MOG peptide with the Seq. ID No. 2 as well as a PLP peptide with the Seq. ID No. 3, for use in the treatment, by topical administration, of multiple sclerosis, wherein said administration decreases the rate of progression of the disease over the year following the end of treatment.

Preferably, a composition according to the present invention is useful in at least one of the following uses:
- lowering a patient's ARR,
- decreasing the rate of development of neurological impairment,
- decreasing the number of newly observed changes as measured by magnetic resonance,
- decreasing the volume of newly observed changes as measured by magnetic resonance,
- decreasing the progression rate of the disease in the year following the end of treatment, wherein preferably the decrease in the rate of progression is based on reducing the number of metastases or decreasing the progression of impaired mobility.

In a preferable embodiment, the indicated peptides are used at a dose of from 1 mg each to 10 mg each.

It unexpectedly turns out that the topical administration of the composition according to the present invention elicits a positive effect in the form of a decrease or inhibition of the development of MS, by inducing a state of immunotolerance against the peptides used, which decreases or eliminates autoreactive lymphocyte activity.

Unexpectedly, it turned out that the desirable medicinal effect is also maintained after the preparation is no longer administered. Unexpectedly, among persons who have previously received the composition according to the present invention, over a year after the therapy has ceased, we have observed a marked decrease in the rate of progression of the disease, consisting in particular of reducing the number of exacerbations as well as decreasing the progression of motor impairment.

The above effects have been confirmed using clinical trials carried out on a group of patients with relapsing-remissive multiple sclerosis.
Fig. 1 shows a graph showing the lymphocyte proliferation level as measure using the mitotic index.
Fig. 2 shows a graph demonstrating the production of interleukin 10
Fig. 3 shows data demonstrating the frequency of occurrence of the exacerbations of the disease multiple sclerosis
Fig. 4 shows an analysis of the rate of progression of neurological impairment on the EDSS scale.

Table 1 contains data relating to the results MRI analysis of the brain.

### Example 1.

We prepared an equimolar solution of a mixture of the MBP peptide (Seq. ID No. 1), MOG (Seq. ID No. 2) and PLP (Seq. ID No. 3) an aqueous solution of physiological saline, which was loaded onto the internal surface of an adhesive bandage. To administer the peptide solution onto the skin we used 3M adhesive hypoallergenic bandages, 3 cm x 5 cm in size. The solution containing the peptide mixture was loaded onto the absorbent portion of a bandage excised from the rest, and then pressed onto the skin and glued on with another, complete bandage.

The bandages were applied in the are of the right arm. for the first 4 weeks the bandages were changed four times (the old one was removed and the new one applied in the same area) once per week, and then monthly until the end of the 12 month.

The study was performed on a group of 30 patients with relapsing-remitting multiple sclerosis (RRMS), with the MS diagnosis based on the McDonald criterion. The inclusion criteria were: age from 18 to 55 years, definite diagnosis of RRMS based on McDonald criteria (20), EDSS scale dysfunction degree from 0 to 5.5, and one or more exacerbations in the preceding year. The patients were excluded from the study if they were treated with steroids in the preceding month, or were treated in the preceding 3 months with interferon beta or glitterier acetate, or if they were ever treated with mitoxanthrone, cyclophosphamide or natalizumab. The study group included 22 women and 8 men aged 36.9±8.0 years. The average duration of MS was 8.3±6.4 years. The entire study and all procedures were approved by the ethics committee of the Medical University of 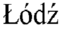 , and all patients gave written consent for the study.

The patients were divided (randomized) into 3 groups:
- Sixteen patients were given a mixture of peptides at a concentration of 1 mg each, in the form of a topical administration on the side of the arm, and covered with an adhesive bandage.
- Four patients were given a mixture of MBP peptide 88-102, MOG 34-54 and PLP 140-152 at a concentration of 10 mg each, in the form of a topical administration on the side of the arm, and covered with an adhesive bandage.

In both cases, the myelin peptides were dissolved in physiological saline and were applied topical in the form and adhesive bandage in the area of the right arm. - Ten patients were given a bandage saturated with physiological saline (placebo).

In the first month of treatment the patients received the peptide mixture or placebo every 7 days, and over the next 11 months once monthly. During treatment, the patients were subjected to regular neurological examinations, the occurrence of exacerbations was recorded, and the patients' state was evaluated according to the Kurtzke scale of neurological impairment (Expanded Disability Status Scale - EDSS). Every 3 months we performed a magnetic resonance imaging (MRI) of patients' heads, in which we evaluated the number and volume of changes in the T2 sequence, as well as the number and volume of changes following the administration of gadoline in sequence T1 (Gd+).

### Magnetic resonance

Resonance studies with intravenous contrasting were performed using a 1.5 Tesla Siemens Avanto Plus scanner. These were performed during the screening visit and repeated every 3 months for one year in each patient. Proton density images and T2 images we used fast spin echo sequences of (TR) 2200-3000 ms, echo times of (TE) 15-50/80-120 ms, echo duration of 4-6ms, a layer thickness of 3 mm and 44 continuous layers. In order to detect changes, we gave the patients intravenous gadoline at a concentration of 0.1mmol/kg and imaged the brain using T1 (TR600-650 ms, TE 10-20ms) with the same layer geometry. Imaging was performed 5 minutes after gadoline administration. Each time patients were positioned in accordance with the line connecting the foremost and lowermost portions of the corpus callosum. Repositioning was performed in accordance with current recommendations. The identification of changes binding gadoline (Gd+), hyperintensive T2 changes and hypointensive T1 changes was performed by two experienced researchers. The total number of Gd+ changes and number of new Gd+ changes were counted, as well as the number of new T2 and T1 changes. Gd+ changes and new T2 and T1 changes are inflammatory change markers. The volume of T2 changes is a measure of the overall damage to the CNS during the progression of the disease. The identified changes were spotted b an experienced neuroradiologist using a semi-quantitative segmentation method, and calculated automatically. The treating neurologist and persons performing the neurological study were blinded to the resonance examination results.

### Results constituting the basis for the evaluation of treatment efficacy

The main result at the basis of the evaluation of the efficacy of treatment consisted of evaluating the accumulated number of active changes, Gd+, per patient and per single scan obtained from every examination during the course of the study. Additional results used in the evaluation of treatment efficacy were: the accumulated number of new T2 changes during all examinations performed during all examinations throughout the year of the study (in months 3,6,9 and 12), the difference in the volume of changes in the T2 and T1 sequences between the beginning and end of the study. The clinical results were used as additional parameters for evaluating treatment efficacy. These were the annual relapse-remission index (ARR), the proportion of patients without disease relapses during the year of the study, as well as changes in the score on the scale of neurological impairment (EDSS) and the proportion of patients who exhibited a progression of impairment on the EDSS scale between the beginning and end of the study.

### Statistical Analysis

The analysis was performed on the comparative results of two groups, placebo vs. the group receiving the mixture of peptides, 1 mg each, or placebo vs. combined groups of myelin peptides, 1 mg and 10 mg each. Statistical analysis was performed using the SPSS package (version 14.0). The normal distribution of the results was evaluated using the Kolmogorov-Smirnovs test. The resonance results were analyzed using the Mann-Whitney U test. The statistical significance level was 0.05.

### Results

### Demographic Data

From among the 30 patients who participated in the study, 10 were randomized into the placebo group, 16 into the to group receiving the myelin peptide mixture at a concentration of 1 mg each and 4 receiving the peptide mixture at a concentration of 10 mg each.

### Safety and Tolerance

The most common side effect was a local skin reaction at the site of administration of the topical adhesive bandage. Moderate redness and itching occurred among 20% (4/20) of patients receiving the topical myelin peptides. No differences were observed among the groups in terms of the occurrence of such side effects as upper respiratory tract infections (n=2) or watering eyes (n=1). No cases of serious adverse side effects occurred. Laboratory blood and urine examinations performed throughout the study showed no significant deviances.

### Magnetic Resonance Results

These results are shown in Table 1.

**Table 1. Comparison of results using MRI**

| **MRI Parameters** | **P placebo** | **1 mg** | **10 mg** | **p*** |
|---|---|---|---|---|
| cnGd+ per patient per slide (+/- SD) | 0.0255 (0.01) | 0.0085 (0.01) | 0.0341 (0.03) | 0.015 |
| Average volume of cnGd(+) (+/- SD) per patient [mm³] | 13,38 (53,59) | 8,4 (16,2) | 23,98 (53,59) | 0.7 |
| Accumulated number of new changes in the T2 sequence per patient(+/- SD) | 2,4 (0.9) | 0.75 (0.25) | 1,25 (0.27) | 0.09 |
| Change of the volume of changes in the T2 sequence per patient [mm³] | 1925,72 (3142,6) | -845,4 (3734,4) | 1265,1 (2055,2) | 0.01 |
| Change of the volume of changes in the T1 sequence per patient [mm³] | 1237,5 (1869,4) | -220.4 (936,0) | -63,2 (1544,8) | 0.01 |

| | | | | |
|---|---|---|---|---|
| cnGd+ = accumulated number of Gd+ changes (+/- SD) = standard deviation All analyses were performed using the Mann-Whitney U test * the p value for a comparison between the 1 mg group and the placebo Treatment through the topical administration of the myelin peptide mixture, at a concentration of 1 mg each, caused a reduction in the occurrence of Gd+ changes by 66.5% per patient per scan in relation to the placebo group (0.0085 vs. 0.0255). This difference was statistically significant (p=0.015). Thus, the main result to be used for evaluating the efficacy of treatment attained statistical significance (Table 1). The advantageous effect of treatment through the topical use of peptides was also confirmed using the remaining results of resonance imaging. The volume of Gd+ in the groups treated with myelin peptides was smaller than in the placebo group (8.4mm³ vs. 13.38 mm³, P=0.07). The number of new T2 changes in the groups treated with myelin peptides was 3 times smaller than in the placebo group (0.75 vs. 2.4, p=0.09). The volume of changes in the T2 sequence following 12 months of treatment decreased in comparison to the onset of treatment in the group treated with myelin peptides by 19.7% (3450.4 vs. 4295.8 mm³) whereas in the placebo group, the volume of T2 changes increased by 25.4% (7580.5 vs. 9506.2 mm³, p=0.01). Likewise, the volume of changes in the T1 sequence in the group treated with myelin peptides was reduced by 14.1% in relation to the onset of the study, whereas in the placebo group the volume of T1 changes increased by 61.2%, (p=0.01). | | | | |

### Clinical Results

In the group of patients treated with a mixture of myelin peptides at a concentration of 1mg each the ARR index was 0.43 7/16) whereas in the placebo group the ARR was 1.4 (14/10. p=0.007). The ARR in the combined groups treated with the mixture of myelin peptides at a concentration of 1 mg and 10 mg each, the ARR was 0.4. The proportion of patients without relapses during the year of the study was significantly higher in the group treated with myelin peptides, 62% (10/16) than in the placebo group 10% (1/10; p=0.0498). During the year-long treatment period in the group treated with myelin peptides 81% (13/16) patients did not show progression of the impairment, whereas in the placebo group only 30% (3/10) patients remained without progression of the impairment (p=0.08). In the combined groups treated with a mixture of myelin peptides at a concentration of 1 mg each and 10 mg each 20% patients worsened on the EDSS scale (4/20) whereas in the placebo group 70% of the patients worsened (7/10) (p=0.16).

### Conclusions:

The results of the clinical trial showed that the use of the mixture of the MBP, MOG and PLP peptides lead to the induction of a state of immunological tolerance as measured by a decreased lymphocyte proliferation isolated from treated patients in relation to these peptides (Fig.1) as well as to the increased production of interleukin 10 (Fig. 2). Moreover, we have observed the appearance of a unique, new population of cells, likely tolerogenic dendritic cells in the lymph nodes of patients treated with myelin peptides.
Patients treated with myelin peptides at a concentration of 1 mg during the year-long treatment had a 65% lower relapse frequency (Fig.3). During the annual treatment period the percent of patients without a relapse in the 1 mg group was 62%, 75% in the 10 mg group, and in the placebo group it was 10%. In terms of the rate of progression of the neurological impairment measured on the EDSS scale, we determined that in patients treated with the mixture of peptides at a concentration of 1 mg, the decrease in the rate of progression of the impairment, changes in the average EDSS score was + 0.08, in relation to the increase of the EDSS score in the placebo group o 0.75 (Fig.4). In the group of patients treated with the peptide at a concentration of 1 mg the percent of patients without progression was 81%, in the 10 mg group 75%, and in the placebo group 30%.

In terms of analyzing the brain magnetic resonance scan (MRI) it was determined that patients treated with the mixture of MBP, MOG and PLP peptides at a concentration of 1 mg over the period of a year had significantly fewer active changes as measured by gadoline (Gd+) binding. The average accumulated number of changes Gd(+) in the groups treated per patient and per slide was 0.0085, whereas in the placebo group it was 0.0255. In patients treated with the peptide at a concentration of 1 mg there was also a decrease in the number of new changes in the T2 sequence in relation to the placebo group, as well as the volume of changes in the T2 sequence and volume of changes in the T1 sequence (Table 1).

The results of the study with the topical administration of three myelin peptides, MBP, MOG and PLP unexpectedly showed a significant reductive effect on disease activity as measured by resonance parameters in patients with relapse-remission multiple sclerosis. The peptide dose used at a concentration of 1 mg each was more effective. However, the small sample size (n=4) of the group treated with the higher dose, 10 mg of each peptide, makes a direct comparison of both doses impossible. For this reason, the efficacy analysis performed compared primarily the group treated with a concentration of 1 mg of each peptide versus the placebo or the combined groups treated with both doses of peptide mixture in comparison to the placebo group. The decreased activity of the disease in resonance analysis was related primarily to a decrease in the number of active gadoline-binding changes, Gd+, in the group treated with the peptide mixture at a concentration of 1mg each in relation to the placebo group (p=0.015) as well as a negative growth in the volume of changes in the T2 sequence (p=0.01) and T1 (p=0.01) between the onset and the end of the study in the group treated with the peptide mixture at a concentration of 1mg. In the placebo group, in accordance to data relating to the natural course of multiple sclerosis, we observed an increase in the volume of changes in both sequences. The remaining resonance parameters also exhibited a strong trend to the advantage of treated with a mixture of myelin peptides. A strong effect of reducing the number of Gd+ changes indicates that topical myelin peptide therapy induces an increase in blood-brain barrier impermeability and a decrease in the generation of new inflammation sites in the brain. The negative growth of the volume of changes in the T1 sequence suggests that topical myelin peptide therapy also decreases the generation of fixed pathological changes in patients' brains. The effect of peptide therapy was markedly evident after 6 months of treatment, which was shown by the decreased number of accumulated Gd+ changes after this period.

Due to the short duration of the study, one year, it should be stressed that patients treated topically with a mixture of myelin peptides had a significantly lower annual relapse index than the placebo group. Likewise, the number of patients without relapses during the treatment period was significantly higher in the group of patients treated with myelin peptides in relation to the placebo. Our study encompassed patients with high disease activity, in the period preceding the study, the annual relapse index was 1.1 in the group treated with the peptide mixture at a concentration of 1mg each, and 1.2 in the placebo group. During the study, the annual relapse index in the placebo group remained high, 1.4, whereas in the group treated with myelin peptides at a concentration of 1 mg it was decreased by 69.3%, and in the combined groups of both peptide concentrations, 1mg and 10 mg, by 71.5% in relation to the placebo group. Moreover, in both groups treated with the mixture of myelin peptides, a significantly higher number of patients exhibited no progression in terms of impairment.

Both doses of myelin peptides used exhibited an excellent safety profile. No serious side effects were observed. Only a small, local dermal reaction occurred in some patients treated topical peptides. These symptoms required no treatment and were self-limiting. The laboratory analyses performed demonstrated no deviations in terms of haematology nor blood chemistry.

It was demonstrated that the use of dermal patches with myelin peptides leads to the formation of an immunotolerance to these peptides. At the same time, it was shown that myelin peptides used topically induced and activation of Langerhans dendritic cells at the site of use, as well as inducing the formation of a unique population of granular dendritic cells in local lymph nodes. In the peripheral portion of the immunological system, the use of topical myelin peptides lead to an increased number of Tr1 regulatory cells which produce interleukin 10, to the decrease in the proliferation in response to the peptides used and to a decrease in the synthesis of interferon gamma and TGF-beta. The resonance and clinical results shown demonstrate the unexpected efficacy of the topical dose of the myelin peptide mixture in therapy of relapsing-remissive multiple sclerosis. This is particularly significant in the light of prior studies on the antigen-specific therapy of multiple sclerosis, wherein there was a dissonance between results relating to the possibility of inducing immunotolerance and the lack of clinical and resonance effects.

All in all, the clinical efficacy and the excellent safety profile documented in this study using the topical administration of three myelin peptides: MBP, MOG and PLP, makes it possible to state that this is a very promising method in terms of the therapy of with relapse-remission as well as other forms of multiple sclerosis. In particular, this type of antigen-specific therapy offers a very precise immunological intervention eliminating the autoimmune mechanisms against myelin peptides, while maintaining the other functionalities of the immune system responsible for the immune protection of the organism.

The pharmaceutical composition used for the treatment of multiple sclerosis (MS) is administered topically, in the form of a patch (adhesive bandage). Although the dose of the pharmaceutical composition according to the present invention may differ depending on disease state, it is important to select appropriate doses. For example, an amount in the range of 1 to 10 mg/patient administered from the moment clinical signs appear until their remission may be used, along with another supporting treatment during the remission period. However, the pharmaceutical composition for the treatment of multiple sclerosis according to the present invention is not limited to such doses.

### Example 2. Remote effects of treatment with a mixture of three myelin peptides in patients with the relapsing-remitting form of multiple sclerosis.

All patients who completed the one-year period of blind clinical testing were then regularly monitored for the next year under open conditions. The patients were examined neurologically using EDSS evaluation, at 3 month intervals by a single neurologist. During the period following the end of the blind study, we registered all exacerbations of the disease meeting the criteria of a relapse.

### A. The relapse activity during the open observation following the blind study.

W grouped 16 patients, who during the study received a mixture of 1 mg of each of the myelin peptides mixture topically. During the period of one year following the study, 3 relapses occurred, 3/16. Thus the ARR for this group was 0.187. All relapses were of moderate intensity, and were treated with intravenous methylprednisolone.

Among the 10 patients, who received the placebo during the blind study, during the year following the end of the study there were three exacerbations which met relapse criteria, 3/10. Thus the ARR in the placebo group was 0.3.

In the group of 4 patients, which received the 10 mg mixture of peptide topically in the blind study, there was one relapse, ¼, and thus the ARR was 0.25 in this group.

Comparing the annual relapse indexes during the one year open observation following the blind study, it turned out that the group treated with the mixture of myelin peptides at a dose of 1 mg, the ARR was significantly lower than in the group of patients receiving the placebo during the blind phase. In the group of patients treated in the blind phase with the mixture of peptides at a dose of 10 mg the ARR during the open observation after the end of treatment was only slightly lower than in patients receiving the placebo. However, this group was very small, only 4 patients, and the annual ARR was significantly increased by the single relapse that occurred in this group.

### B. Progression of the impairment during the period of open observation following the termination of the blind study.

In the group of 16 patients, who during the blind study received the 1mg myelin peptide mixture topically, during the one year period after the end of the study, there was a deterioration of impairment using the EDSS scale in 2 patients in a 3 month period, 2/16. In both cases the EDSS score grew by 0.5 pts. Thus, in the entire 16-patient group, patients treated during the blind phase with a mixture of peptides at a dose of 1mg, the average increase in the EDSS score in the year following the study was 0.0625.

W group the group of 10 patients, who received the placebo during the blind phase, in the year following the end of the study, in 3 patients there was a confirmed deterioration of impairment over a 3 month period as determined on the EDSS scale, 3/10. The individual EDSS score increases were, +0.5 pts., +1.0 pts. and +1.0 pts. Thus, in the entire 10 person group of patients receiving the placebo during the bland phase, the average EDSS increase was 0.25.

In the group of 4 patients, who received the 10 mg peptide mixture peptide topically, during the year following the end of the study there was a single confirmed deterioration confirmed by the EDSS score of 1.5 pts. Thus, the average EDSS score increase in this group was 0.375.

Comparing the progression of the impairment as measured by the EDSS scale during the period of the year-long open observation, it turned out that in the group treated with a mixture of myelin peptides at a dose of 1 mg, the number of patients with a fixed progression of the impairment, as well as the average EDSS score increase were significantly lower than in the group of patients receiving the placebo in the blind phase. In the group of patients treated in the blind phase with a mixture of peptides at a dose of 10 mg, the number of patients with a permanent deterioration as well as the average increase of the EDSS score during the open observation following the end of the study was comparable to that of the patients previously receiving the placebo. However, this group was very small, only 4 patients, and the annual impairment progression was heavily weighed by the one relapsing patient.

To summarise the results of the delayed treatment results observed during the year following the end of the administration of the preparation, it should be stated that in patients who received the preparation according to the present invention unexpectedly we observed a decreased rate of progression of the disease over the year following the end of treatment. The observed signs of this effect are a reduced number of relapses as well as a decreased progression of the motor impairment.

## Claims

1. A pharmaceutical composition consisting of: a MBP peptide with the Seq. ID No. 1, a MOG peptide with the Seq. ID No. 2 as well as a PLP peptide with the Seq. ID No. 3, for use in the treatment, by topical administration, of multiple sclerosis, wherein said administration decreases the rate of progression of the disease over the year following the end of treatment.

2. The pharmaceutical composition for use according to Claim 1, wherein said administration decreases the annualized relapse rate (ARR) in patients.

3. The pharmaceutical composition according to Claim 1, wherein said administration decreases the rate of development of neurological impairment.

4. The pharmaceutical composition for use according to Claim 1, wherein said administration decreases the number of new changes observed in patients as measured by magnetic resonance.

5. The pharmaceutical composition for use according to Claim 1, wherein said administration decreases the volume of new changes observed in patients as measured by magnetic resonance.

6. The pharmaceutical composition for use according to Claim 1, wherein the decrease in the rate of progression of the disease is based on reducing the number of relapses.

7. The pharmaceutical composition for use according to Claim 1, wherein the decrease in the rate of progression of the disease is based on a decrease in the progression of motor impairment.

## Patentansprüche

1. Pharmazeutische Zusammensetzung bestehend aus einem MBP-Peptid mit der SEQ ID Nr. 1, einem MOG-Peptid mit der SEQ ID Nr. 2 sowie einem PLP-Peptid mit der SEQ ID Nr. 3 zur Verwendung bei der Behandlung von multipler Sklerose durch topische Verabreichung, wobei die Verabreichung die Geschwindigkeit des Fortschreitens der Krankheit über das Jahr nach Ende der Behandlung senkt.

2. Pharmazeutische Zusammensetzung zur Verwendung gemäß Anspruch 1, wobei die Verabreichung die annualisierte Rezidivrate (ARR) bei Patienten senkt.

3. Pharmazeutische Zusammensetzung zur Verwendung gemäß Anspruch 1, wobei die Verabreichung die Geschwindigkeit der Entwicklung der neurologischen Beeinträchtigung senkt.

4. Pharmazeutische Zusammensetzung zur Verwendung gemäß Anspruch 1, wobei die Verabreichung die Anzahl der neuen Veränderungen senkt, die man bei Patienten bei der Messung durch magnetische Resonanz beobachtet.

5. Pharmazeutische Zusammensetzung zur Verwendung gemäß Anspruch 1, wobei die Verabreichung das Volumen der neuen Veränderungen senkt, die man bei Patienten bei der Messung durch magnetische Resonanz beobachtet.

6. Pharmazeutische Zusammensetzung zur Verwendung gemäß Anspruch 1, wobei die Abnahme der Geschwindigkeit des Fortschreitens der Krankheit auf einer Reduktion der Anzahl der Rezidive beruht.

7. Pharmazeutische Zusammensetzung zur Verwendung gemäß Anspruch 1, wobei die Abnahme der Geschwindigkeit des Fortschreitens der Krankheit auf einer Abnahme des Fortschreitens der motorischen Beeinträchtigung beruht.

## Revendications

1. Composition pharmaceutique constituée de: un peptide MBP ayant la SEQ ID NO : 1, un peptide MOG ayant la SEQ ID NO : 2 ainsi qu'un peptide PLP ayant la SEQ ID NO : 3, pour son utilisation dans le traitement, par administration topique, de la sclérose en plaques, dans laquelle ladite administration diminue le taux de progression de la maladie au cours de l'année suivant la fin du traitement.

2. Composition pharmaceutique pour son utilisation selon la revendication 1, dans laquelle ladite administration diminue le taux de rechute annualisé (TRA) chez les patients.

3. Composition pharmaceutique selon la revendication 1, dans laquelle ladite administration diminue le taux de développement de détérioration neurologique.

4. Composition pharmaceutique pour son utilisation selon la revendication 1, dans laquelle ladite administration diminue le nombre de nouveaux changements observés chez des patients, comme mesuré par résonance magnétique.

5. Composition pharmaceutique pour son utilisation selon la revendication 1, dans laquelle ladite administration diminue le volume de nouveaux changements observés chez les patients, comme mesuré par résonance magnétique.

6. Composition pharmaceutique pour son utilisation selon la revendication 1, dans laquelle la diminution du taux de progression de la maladie est basée sur la réduction du nombre de rechutes.

7. Composition pharmaceutique pour son utilisation selon la revendication 1, dans laquelle la diminution du taux de progression de la maladie est basée sur une diminution de la progression de la détérioration motrice.
